# EUROPEAN PATENT APPLICATION

(11) **EP 3 912 684 A1**
(43) Date of publication of application: **24.11.2021**
(21) Application number: 19898550.9
(22) Date of filing: 16.12.2019
(51) Int. Cl.: A61Q 19/00, B82Y 30/00, A23L 33/115, A61K 8/04, A61K 8/34, A61K 8/37, A61K 8/39, A61K 8/55, A61K 8/63, A61K 8/68

(54) **CERAMIDE DISPERSION COMPOSITION**

(30) Priority: 18.12.2018 JP 2018236171
(71) Applicant: Genuine R&D Co., Ltd., Fukuoka-shi, Fukuoka 811-0215 (JP)
(72) Inventor: ARAKAWA, Jun, Soraku-gun, Kyoto 619-0237 (JP); KAMIYA, Naoki, Soraku-gun, Kyoto 619-0237 (JP); HIRAKI, Shinobu, Fukuoka-shi, Fukuoka 811-0215 (JP); MIYANABE, Masakatsu, Tokyo 105-0014 (JP)
(74) Representative: Müller-Boré & Partner Patentanwälte PartG mbB
(86) International application number: PCT/JP2019/049253
(87) International publication number: WO 2020/129920

(57) **Abstract**

Provided is a method for stably dispersing a naturally occurring free ceramide (in particular, a naturally occurring human-type free ceramide) containing a long-chain fatty acid in the ceramide skeleton in an aqueous phase.

More specifically, provided is a ceramide dispersion composition comprising a naturally occurring free ceramide, a nonionic surfactant, a phospholipid, and a C₂₋₆ diol, the mass ratio of the naturally occurring free ceramide to the diol being 1:3 to 25, the naturally occurring free ceramide comprising a free ceramide that contains a fatty acid having 20 or more carbon atoms in the ceramide skeleton thereof.

## Description

### Technical Field

The present invention relates to a ceramide dispersion composition and the like.

### Background Art

We human beings have a stratum corneum formed as the outermost epidermal layer to counteract the stress of dryness on the ground. The stratum corneum is composed of dead cells that have lost their nuclei and of intercellular lipids. In recent years, it is widely known in the field of dermatology that the formation of intercellular lipids is extremely important for skin barrier formation. Among intercellular lipids, free ceramides play a particularly important role. It is said that free ceramides decrease with aging or by stress and that it is important to supply them by external application to maintain the normal barrier. For this purpose, cosmetics and external preparations containing ceramides are commercially available. In particular, there is a great demand for human-type free ceramides (free ceramides present in human skin) because they are preferred as ceramides to be applied to humans.

The human-type free ceramides contained in such commercial available cosmetics and external preparations are synthetic products. The synthetic products have a basic skeleton similar to that of free ceramides in human skin; however, in the synthetic products, the carbon chain length of the sphingoid base and fatty acid portion (especially fatty acid portion) constituting the basic skeleton is relatively short (e.g., about C₁₈). In contrast, many of the free ceramides actually present in human skin have a carbon chain that is relatively long (e.g., about C₂₂ to C₂₆). Although free ceramides contained in conventional commercially available cosmetics and external preparations are called "human-type free ceramide," their composition greatly differs from that of free ceramides actually present in human skin. In particular, it is commonly known that long-chain ceramides have higher barrier performance, and it is desirable to make the carbon chains of synthetic human-type free ceramides longer to make them as close as possible to the carbon chain length of ceramides in human skin. However, this is not easy in terms of synthesis technology.

### Citation List

### Patent Literature

PTL 1: JP2012-126910A
PTL 2: JP2004-331595A
PTL 3: JP2015-174840A
PTL 4: JP2013-224314A

### Non-patent Literature

NPL 1: Fragrance Journal, December 2013, pp. 44-49

### Summary of Invention

### Technical Problem

It has been recently found that free ceramide extracts from brewing lees, which mainly comprise many ceramides containing C₂₄ and C₂₆ long-chain fatty acids attached to phytosphingosine, have a carbon chain length greater than that of synthetic free ceramides of the same type, and thus more closely resemble free ceramides that are present in human skin (Patent Literature 1 and Non-patent Literature 1).

However, the present inventors found that such human-type free ceramides that occur in nature (naturally occurring human-type free ceramides) are extremely difficult to disperse in an aqueous phase compared with conventional synthetic human-type free ceramides, making it difficult to use them as external preparations for the skin, such as cosmetics. The inventors also found that naturally occurring human-type free ceramides are superior to synthetic human-type free ceramides in terms of effects (e.g., skin barrier effect). That is, the inventors found that although naturally occurring human-type free ceramides are superior in terms of effects, it is difficult to actually use them as, for example, cosmetics and external preparations because of their poor dispersibility.

Regarding the methods for dispersing synthetic human-type free ceramides, the method for dispersing ceramide NDS (also referred to as "ceramide 2") is disclosed in, for example, Patent Literature 2, and the method for dispersing ceramide AP or ceramide NP is disclosed in, for example, Patent Literature 3. Moreover, Patent Literature 4 discloses a method for obtaining a nano-dispersion of ceramides by dissolving an oily component containing ceramide AP and ceramide NP in a water-soluble organic solvent to prepare an oil phase, allowing the obtained oil phase and an aqueous phase to separately pass through a microchannel, and then instantaneously mixing them by counter-flow collision. However, it is difficult to stably disperse naturally occurring human-type free ceramides in an aqueous phase even by the conventional methods described above.

A primary object of the present invention is to provide a method for stably dispersing a naturally occurring free ceramide (in particular, a naturally occurring human-type free ceramide) containing a long-chain fatty acid in the ceramide skeleton in an aqueous phase.

### Solution to Problem

The present inventors found the possibility that a naturally occurring human-type free ceramide can be stably dispersed in an aqueous phase by using the free ceramide in combination with a nonionic surfactant, a phospholipid, and a polyhydric alcohol. The inventors conducted further research and accomplished the present invention.

The present invention includes, for example, the subject matter described in the following items.

### Item 1.

A ceramide dispersion composition comprising a naturally occurring free ceramide, a nonionic surfactant, a phospholipid, and a C₂₋₆ diol,
the mass ratio of the naturally occurring free ceramide to the diol being 1:3 to 25,
the naturally occurring free ceramide comprising a free ceramide that contains a fatty acid having 20 or more carbon atoms in the ceramide skeleton thereof.

### Item 2.

The ceramide dispersion composition according to Item 1, wherein 90 mass% or more of the naturally occurring free ceramide is a free ceramide in which the number of carbon atoms constituting a molecule is 40 or more, and 60 mass% or more of the naturally occurring free ceramide is a free ceramide in which the number of carbon atoms constituting a molecule is 42 or more. Item 3.

The ceramide dispersion composition according to Item 1 or 2, wherein 60 mass% or more of the naturally occurring free ceramide is ceramide AP and ceramide NP.

### Item 4.

The ceramide dispersion composition according to any one of Items 1 to 3, wherein 80 mass% or more of the naturally occurring free ceramide is ceramide AP.

### Item 5.

The ceramide dispersion composition according to any one of Items 1 to 4, wherein the naturally occurring free ceramide is at least one free ceramide selected from the group consisting of
free ceramides with a ceramide skeleton that is a combination of a sphingoid base containing 3 hydroxyl groups, 18 carbon atoms, and 0 or 1 carbon-carbon double bond; and a fatty acid containing 22 to 26 carbon atoms, 0 carbon-carbon double bonds, and 0, 1, or 2 hydroxyl groups, and
free ceramides with a ceramide skeleton that is a combination of a sphingoid base containing 3 hydroxyl groups, 20 carbon atoms, and no carbon-carbon double bonds; and a fatty acid containing 24 or 25 carbon atoms, 0 carbon-carbon double bonds, and 0, 1, or 2 hydroxyl groups.

### Item 6.

The ceramide dispersion composition according to any one of Items 1 to 5, wherein the C₂₋₆ diol is at least one diol selected from the group consisting of propylene glycol, butylene glycol, diethylene glycol, and pentylene glycol.

### Item 7.

The ceramide dispersion composition according to any one of Items 1 to 6, wherein the phospholipid is at least one phospholipid selected from the group consisting of glycerophospholipids and sphingophospholipids.

### Item 8.

The ceramide dispersion composition according to any one of Items 1 to 7, wherein 50 mass% or more of the phospholipid is phosphatidylcholine.

### Item 9.

The ceramide dispersion composition according to any one of Items 1 to 8, wherein the nonionic surfactant is a polyglycerol mono-fatty acid ester.

### Items 10.

The ceramide dispersion composition according to any one of Items 1 to 9, further comprising a sterol compound.

### Item 11.

The ceramide dispersion composition according to any one of Items 1 to 10, further comprising a C₁₀₋₂₄ saturated or unsaturated fatty acid.

### Item 12.

The ceramide dispersion composition according to any one of Items 1 to 11, wherein particles containing a naturally occurring human-type free ceramide and dispersed in the composition have an average particle size of 100 nm or less. Item 13.

The ceramide dispersion composition according to any one of Items 1 to 12, which is a cosmetic composition or a food composition.

### Advantageous Effects of Invention

A naturally occurring free ceramide containing a long-chain fatty acid in the ceramide skeleton can be stably dispersed in an aqueous phase.

### Brief Description of Drawings

Fig. 1 shows the analysis results of the free ceramide species contained in a ceramide composition (naturally occurring free ceramide) through analysis of the ceramide composition by LC-MS (high-performance liquid chromatography-mass spectrometry).
Fig. 2 shows the structure of ceramide III, ceramide VI, and ceramide NDS.

### Description of Embodiments

Embodiments encompassed by the present invention are described in more detail below. The present invention preferably includes a ceramide dispersion composition, a method for producing the composition, etc.; however, the present invention is not limited to these. The present invention encompasses all that is described in the present specification and can be recognized by those skilled in the art.

The ceramide dispersion composition encompassed by the present invention comprises a naturally occurring free ceramide, a nonionic surfactant, a phospholipid, and a C₂₋₆ diol. Below, the ceramide dispersion composition encompassed by the present invention may be referred to as the "ceramide dispersion composition of the present invention." In the ceramide dispersion composition, the naturally occurring free ceramide is preferably dispersed. As is described in detail below, the naturally occurring free ceramide used in the ceramide dispersion composition of the present invention is a specific free ceramide containing a long-chain fatty acid in the ceramide skeleton, particularly preferably a naturally occurring human-type free ceramide or a similar free ceramide.

The naturally occurring free ceramide can be rephrased as a "free ceramide derived from an organism." The organism is not particularly limited, and examples include animals, plants, fungi, and the like. However, it is preferred that humans are excluded. The phrase "free ceramide derived from an organism" as used herein encompasses not only a free ceramide present in tissue of an organism, but also a free ceramide produced by modifying biological tissue by an organism of the same species or a different species. The modification is preferably, for example, fermentation. Preferred examples of the naturally occurring free ceramide include free ceramides extracted from brewing food lees, and more specific preferred examples include free ceramides extracted from soy sauce lees. Such naturally occurring free ceramides can be prepared, for example, by the method disclosed in Patent Literature 1.

A ceramide is a compound having a structure (-NH-CO-) in which the carboxyl group (-COOH) of a fatty acid is attached to the amino group (-NH₂) of a sphingoid base. Polar groups such as sugar and phosphoric acid are further attached to an alcoholic hydroxyl group (-OH) of the sphingoid base of ceramides to form a sphingoglycolipid and a sphingophospholipid, respectively. A ceramide to which sugar is attached is called, in particular, a "glycosylceramide"; and, in particular, when the sugar is glucose, it is called "glucosylceramide." A ceramide to which no sugar or phosphoric acid is attached is called, in particular, a "free ceramide."

In addition, free ceramides are generally intermediate metabolites of a sphingolipid synthesis system and are thus present in only trace amounts, especially in animals and plants. Ceramides extracted from animals and plants are usually those in which sugar or phosphoric acid is attached to ceramide, such as galactosylceramide, glucosylceramide, and sphingomyelin, and these ceramide derivatives (sphingoglycolipids and sphingophospholipids) have long been used under the name "natural ceramide" in cosmetics and health foods. However, the orientation of these ceramide derivatives in intercellular lipids is completely different from that of free ceramides because of the attachment of sugar or phosphoric acid. Thus, the ceramide derivatives are less effective than free ceramides in terms of, e.g., skin barrier properties.

The naturally occurring free ceramide contained in the ceramide dispersion composition of the present invention may be a single free ceramide or a combination of two or more free ceramides. The phrase "naturally occurring free ceramide" as used herein is intended to include both a single free ceramide and a combination of two or more free ceramides.

The sphingoid base constituting the free ceramide used in the ceramide dispersion composition of the present invention preferably has 2 or 3 hydroxyl groups, and more preferably 3 hydroxyl groups. Further, the sphingoid base preferably has 14 to 22 (14, 15, 16, 17, 18, 19, 20, 21, or 22) carbon atoms, more preferably 16 to 20 carbon atoms, and even more preferably 18 or 20 carbon atoms. The sphingoid base also preferably has 0 or 1 carbon-carbon double bond. More specific examples of preferable sphingoid bases include sphingosine, dihydrosphingosine, phytosphingosine, and the like. Of these, phytosphingosine is particularly preferable.

The fatty acid constituting the free ceramide used in the ceramide dispersion composition of the present invention has 20 or more carbon atoms, preferably 20 to 30 (20, 21, 22, 23, 24, 25, 26, 27, 28, 29, or 30) carbon atoms, more preferably 20 to 28 carbon atoms, even more preferably 20 to 26 carbon atoms, still even more preferably 22 to 26 carbon atoms, and particularly preferably 24 or 26 carbon atoms. The fatty acid also preferably has 0 or 1 carbon-carbon double bond, and more preferably 0 carbon-carbon double bonds (i.e., a saturated fatty acid). The fatty acid also preferably has 0, 1, or 2 hydroxyl groups, and more preferably 1 or 2 hydroxyl groups. Although there is no particular limitation, when the fatty acid contains hydroxyl, the hydroxyl is preferably α-hydroxyl.

The combination of the sphingoid base and the fatty acid in the ceramide skeleton of the free ceramide may be any combination of sphingoid bases and fatty acids described above. Of these, a preferable example of the combination is a combination of a sphingoid base containing 2 or 3 (in particular, 3) hydroxyl groups and a fatty acid that does not contain or contains hydroxyl (particularly preferably α-hydroxyl when it contains hydroxyl). When the fatty acid contains hydroxyl, the number of hydroxyl groups is preferably 0, 1, or 2, and more preferably 1 or 2.

Preferable examples include ceramide AP, which is a combination of phytosphingosine (P) and a fatty acid containing 1 hydroxyl group (preferably α-hydroxyl group) (A); ceramide NP, which is a combination of phytosphingosine (P) and a fatty acid containing 0 hydroxyl groups (N); and the like. Further, ceramide DP, which is a combination of phytosphingosine (P) and a fatty acid containing 2 hydroxyl groups (D), is also a preferable example. "Ceramide AP" and "ceramide NP" are terms commonly used in the art; however, "ceramide DP" is a term that is used in the present specification, and is not a commonly used term. Specific examples of ceramide DP include dihydroxylignoceroyl phytosphingosine and the like.

Among these, (i) free ceramides with a ceramide skeleton that is a combination of a sphingoid base containing 3 hydroxyl groups, 18 carbon atoms, and 0 or 1 carbon-carbon double bond; and a fatty acid containing 22 to 26 carbon atoms, 0 carbon-carbon double bonds, and 0, 1, or 2 hydroxyl groups, and (ii) free ceramides with a ceramide skeleton that is a combination of a sphingoid base containing 3 hydroxyl groups, 20 carbon atoms, and no carbon-carbon double bonds; and a fatty acid containing 24 or 25 carbon atoms, 0 carbon-carbon double bonds, and 0, 1, or 2 hydroxyl groups are preferable as free ceramides. Of these, specific examples of preferable free ceramides include t18:0-22:0h, t18:1-22:0h, t18:0-23:0h, tl8:1-23:0h, t18:0-24:0h, t18:1-24:0h, t20:0-24:0h, t18:1-26:0h, t18:0-25:0h, t18:0-24:0h₂, and the like. This notation is explained using "t18:0-22:0h" as an example. The first half ("t18:0") is information about the sphingoid base, and indicates a sphingoid base that contains 3 hydroxyl groups ("t"), 18 carbon atoms, and 0 carbon-carbon double bonds (i.e., containing no carbon-carbon double bonds). The latter half ("22:0h") is information about the fatty acid, and indicates a fatty acid that contains 22 carbon atoms, 0 carbon-carbon double bonds, and 1 hydroxyl group ("h"). "h₂" indicates that the fatty acid contains 2 hydroxyl groups.

Among the specific free ceramides, t18:0-24:0h, t18:1-24:0h, and t20:0-24:0h are particularly preferable.

Moreover, the ceramide dispersion composition of the present invention preferably comprises at least t18:0-24:0h, t18:1-24:0h, and t20:0-24:0h as free ceramides. It is more preferable that the total amount of t18:0-24:0h, t18:1-24:0h, and t20:0-24:0h is 30 mass% or more, 40 mass% or more, or 50 mass% or more of the total amount of free ceramides contained in the composition.

The free ceramides may be used singly or in a combination of two or more.

As the naturally occurring free ceramide, those in which the number of carbon atoms constituting a free ceramide molecule is 40 or more are preferable, and those in which the number of carbon atoms constituting a free ceramide molecule is 42 or more are more preferable. The upper limit of the number of carbon atoms is not particularly limited and is preferably, for example, 46 or less. The number of carbon atoms is preferably 40 to 46 (40, 41, 42, 43, 44, 45, or 46), and more preferably 42 to 44. Free ceramide(s) in which the number of carbon atoms constituting a molecule is 40 or more preferably make up 90 mass% or more, more preferably 91, 92, 93, 94, 95, 96, 97, or 98 mass% or more, of the total free ceramide(s) forming the naturally occurring free ceramide. It is more preferable that free ceramide(s) in which the number of carbon atoms constituting a molecule is 42 or more make up 60 mass% or more, more preferably 61, 62, 63, 64, 65, 66, 67, 68, 69, 70, 71, 72, 73, 74, 75, 76, 77, 78, 79, 80, 81, 82, 83, 84, 85, 86, or 87 mass% or more. It is further preferable that free ceramide(s) in which the number of carbon atoms constituting a molecule is 40 or more make up 90 mass% or more and that free ceramide(s) in which the number of carbon atoms constituting a molecule is 42 or more make up 60 mass% or more.

It is also preferable that the total of ceramide AP and ceramide NP is 60 mass% or more of the total free ceramides forming the naturally occurring free ceramide. It is more preferable that the total of ceramide AP and ceramide NP is 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, or 94 mass% or more of the total free ceramides forming the naturally occurring free ceramide.

Ceramide AP also preferably makes up 40 mass% or more, and more preferably 45, 50, 55, 60, 65, 70, 75, 80, 85, or 90 mass% or more of the total free ceramides forming the naturally occurring free ceramide. Ceramide NP also preferably makes up 0.5 mass% or more, and more preferably 1 mass% or more, of the total free ceramides forming the naturally occurring free ceramide.

Moreover, although there is no particular limitation, it is also preferable that phytoceramide(s) make up 90 mass% or more of the total free ceramide(s) forming the naturally occurring human-type free ceramide.

Each ceramide can be prepared by a known method, or a method easily conceivable from a known method. Further, commercially available ceramides can also be purchased and used as the naturally occurring free ceramide used in the present invention. For example, natural human-type ceramide sold as an external cosmetic material by Genuine R&D Co., Ltd., which contains many ceramide species preferable for use in the composition according to the present invention, is suitable. Moreover, for example, a ceramide composition comprising one or more ceramide species suitable for the ceramide dispersion composition of the present invention can be obtained by the method disclosed in Patent Literature 1 described above.

Preferred examples of the C₂₋₆ diol used in the ceramide dispersion composition of the present invention include ethylene glycol, propylene glycol, 1,2-butanediol, 1,3-butanediol, 1,4-butanediol, 1,2-pentanediol, 1,5-pentanediol, 2,4-pentanediol, 1,2-hexanediol, 1,6-hexanediol, diethylene glycol, triethylene glycol, and the like. Propylene glycol, 1.3-butanediol, diethylene glycol, and pentylene glycol are more preferable in terms of a skin care feeling when the composition is used as a cosmetic. The C₂₋₆ diols may be used singly or in a combination of two or more. Although there is no particular limitation, the C₂₋₆ diol can be preferably used as a solvent for first dissolving the naturally occurring free ceramide when the ceramide dispersion composition of the present invention is prepared.

The ceramide dispersion composition of the present invention comprises the C₂₋₆ diol in an amount of 3 to 25 parts by mass, per part by mass of the naturally occurring free ceramide. The lower limit of this range may be 4, 5, 6, 7, 8, 9, 10, 11, 12, 13, 14, 15, 16, 17, 18, or 19 parts by mass. The upper limit of this range may be 24, 23, 22, or 21 parts by mass. This range is more preferably 3 to 20 parts by mass. When the amount of the C₂₋₆ diol is 3 parts by mass or more, the naturally occurring free ceramide can be efficiently dissolved, which makes problems caused by the process proceeding to an emulsification and dispersion step with minute insoluble matter remaining, such as difficulty in reducing the size of the particles and crystal growth during storage, less likely to occur. When the amount of the C₂₋₆ diol is 25 parts by mass or less, it is unlikely that aggregation of particles is promoted during storage, causing cloudiness and precipitation.

As described above, the ceramide dispersion composition of the present invention further comprises a nonionic surfactant and a phospholipid. Since the composition further comprises a nonionic surfactant and a phospholipid, the composition comprises finer dispersed particles and can produce a more excellent skin-care effect when used for an external preparation for the skin.

Examples of nonionic surfactants include propylene glycol fatty acid esters, glycerol fatty acid esters, polyglycerol fatty acid esters, polyoxyethylene fatty acid esters, sorbitan fatty acid esters, polyoxyethylene hydrogenated castor oil, polyoxyethylene sterols, polyoxyethylene alkyl ethers, polyoxyethylene alkyl esters, polyethylene glycol fatty acid esters, sucrose fatty acid esters, and the like. Polyglycerol fatty acid esters are particularly preferable in terms of reduction in the size of the particles and small temperature dependence. Of polyglycerol fatty acid esters, polyglycerol mono-fatty acid esters are particularly preferable.

The properties of nonionic surfactants are sometimes expressed by using the hydrophilic-lipophilic balance (HLB). In the present invention, a compound with an HLB value between 8 and 20 is used. A compound with an HLB value of 10 to 16 is particularly preferable.

The polyglycerol fatty acid ester is preferably at least one ester of a polyglycerol with an average degree of polymerization of 10 and a C₈₋₁₈ fatty acid, such as caprylic acid, capric acid, lauric acid, myristic acid, palmitic acid, stearic acid, oleic acid, or linoleic acid.

Preferred examples of such polyglycerol fatty acid esters include hexaglycerol monooleate, hexaglycerol monopalmitate, hexaglycerol monomyristate, hexaglycerol monolaurate, decaglycerol monooleate, decaglycerol monostearate, decaglycerol monopalmitate, decaglycerol monomyristate, decaglycerol monolaurate, and the like.

Of the polyglycerol fatty acid esters, at least one member selected from the group consisting of decaglycerol monolinoleate, decaglycerol monooleate, decaglycerol monoisostearate, decaglycerol monopalmitate, decaglycerol monomyristate, and decaglycerol monolaurate is more preferable, and decaglycerol monooleate is particularly preferable.

A combination of one polyglycerol fatty acid ester selected from polyglycerol fatty acid esters with an HLB of 10 to 16, and one or more polyglycerol fatty acid esters whose molecular structure is different therefrom and that are selected from polyglycerol fatty acid esters with an HLB of 5 to 15 may be used as the polyglycerol fatty acid ester. The polyglycerol fatty acid esters with an HLB of 5 to 15 may be polyglycerol fatty acid esters included in the polyglycerol fatty acid esters described above, or other polyglycerol fatty acid esters.

The polyglycerol fatty acid esters may be commercially available products.

Examples of commercially available products of the polyglycerol fatty acid esters include NIKKOL DGMS, NIKKOL DGMO-CV, NIKKOL DGMO-90V, NIKKOL DGDO, NIKKOL DGMIS, NIKKOL DGTIS, NIKKOL Tetraglyn 1-SV, NIKKOL Tetraglyn 1-O, NIKKOL Tetraglyn 3-S, NIKKOL Tetraglyn 5-S, NIKKOL Tetraglyn 5-O, NIKKOL Hexaglyn 1-L, NIKKOL Hexaglyn 1-M, NIKKOL Hexaglyn 1-SV, NIKKOL Hexaglyn 1-O, NIKKOL Hexaglyn 3-S, NIKKOL Hexaglyn 4-B, NIKKOL Hexaglyn 5-S, NIKKOL Hexaglyn 5-0, NIKKOL Hexaglyn PR-15, NIKKOL Decaglyn 1-L, NIKKOL Decaglyn 1-LEX, NIKKOL Decaglyn 1-M, NIKKOL Decaglyn 1-MEX, NIKKOL Decaglyn 1-SV, NIKKOL Decaglyn 1-SVEX, NIKKOL Decaglyn 1-50SV, NIKKOL Decaglyn 1-ISV, NIKKOL Decaglyn 1-ISVEX, NIKKOL Decaglyn 1-O, NIKKOL Decaglyn 1-OV, NIKKOL Decaglyn 1-OVEX, NIKKOL Decaglyn 1-LN, NIKKOL Decaglyn 2-SV, NIKKOL Decaglyn 2-ISV, NIKKOL Decaglyn 3-SV, NIKKOL Decaglyn 3-OV, NIKKOL Decaglyn 5-SV, NIKKOL Decaglyn 5-HS, NIKKOL Decaglyn 5-IS, NIKKOL Decaglyn 5-OV, NIKKOL Decaglyn 5-O-R, NIKKOL Decaglyn 7-S, NIKKOL Decaglyn 7-0, NIKKOL Decaglyn 10-SV, NIKKOL Decaglyn 10-IS, NIKKOL Decaglyn 10-OV, NIKKOL Decaglyn 10-MAC, and NIKKOL Decaglyn PR-20, all of which are produced by Nikko Chemicals Co., Ltd.; Ryoto Polyglyester L-7D, L-10D, M-10D, P-8D, SWA-10D, SWA-15D, SWA-20D, S-24D, S-28D, O-15D, O-50D, B-70D, B-100D, ER-60D, LOP-120DP, DS13W, DS3, HS11, HS9, TS4, TS2, DL15, and DO13, all of which are produced by Mitsubishi-Chemical Foods Corporation; Sunsoft Q-17UL, Sunsoft Q-14S, and Sunsoft A-141C, all of which are produced by Taiyo Kagaku Co., Ltd.; Poem DO-100 and Poem J-0021, both of which are produced by Riken Vitamin Co., Ltd.; S Face IS-201P, IS-401P, IS-601P, IS-1001P, M-1001P, O-201P, O-401P, O-601P, and O-1001P, all of which are produced by Sakamoto Yakuhin Kogyo Co., Ltd.; and the like.

Of these, preferred examples of commercially available products of the polyglycerol fatty acid esters include NIKKOL Decaglyn 1-L, NIKKOL Decaglyn 1-LEX, NIKKOL Decaglyn 1-M, NIKKOL Decaglyn 1-MEX, NIKKOL Decaglyn 1-OV, NIKKOL Decaglyn 1-OVEX, NIKKOL Decaglyn 1-ISV, NIKKOL Decaglyn 1-ISVEX, NIKKOL Decaglyn 1-LN, and Ryoto Polyglyester L-7D, L-10D, M-10D, P-8D, SWA-10D, SWA-15D, SWA-20D, S-24D, S-28D, O-15D, O-50D, B-70D, B-100D, ER-60D, LOP-120DP, IS-1001P, and O-1001P.

The ceramide dispersion composition of the present invention may comprise a single nonionic surfactant or a combination of two or more nonionic surfactants.

The content of the nonionic surfactant in the ceramide dispersion composition of the present invention is preferably 0.1 to 30 parts by mass, per part by mass of the naturally occurring free ceramide, in terms of reduction in the size of the dispersed particles and a skin care effect when the ceramide dispersion composition is used for cosmetics or the like. The lower limit of this range may be 0.5, 1, 1.5, 2, or 2.5 parts by mass. The upper limit of this range may be 25, 20, 15, 10, 5, 4.5, or 4 parts by mass. This range is more preferably 0.5 to 20 times, and even more preferably 1 to 10 times the amount of the naturally occurring free ceramide.

As the phospholipid, both glycerophospholipids and sphingophospholipids may be preferably used.

Glycerophospholipids, which contain hydrophilic and hydrophobic groups in their molecules, have been widely used as emulsifiers in the fields of food, pharmaceuticals, cosmetics, and the like. Glycerophospholipids commonly used in the fields of food and cosmetics are mixtures of several types of glycerophospholipids, and these mixtures are called "lecithin." Preferred examples of glycerophospholipids used in the ceramide dispersion composition of the present invention include lecithin.

Lecithin with a purity of 60% or more is industrially used as purified lecithin, and such purified lecithin may be used in the ceramide dispersion composition of the present invention. In order to form finer dispersed particles, lecithin with a purity of 80% or more, which is commonly referred to as "high-purity lecithin," is preferable, and lecithin with a purity of 90% or more is more preferable.

Examples of lecithin suitable for use include various known lecithins extracted and separated from organisms such as plants, animals, and microorganisms. Specific examples of such lecithin include various lecithins derived from plants, such as soybeans, corn, sunflower seeds, peanuts, rapeseed, and wheat, animals, such as egg yolk and cows, and microorganisms, such as *Escherichia coli.*

Examples of names of compounds as glycerophospholipids contained in such lecithin include phosphatidic acid, phosphatidylglycerol, phosphatidylinositol, phosphatidylethanolamine, phosphatidylmethylethanolamine, phosphatidylcholine, phosphatidylserine, bisphosphatidic acid, diphosphatidylglycerol, and the like.

In addition to the above high-purity lecithin, it is also possible to use hydrogenated lecithin, enzymatically decomposed lecithin, enzymatically decomposed hydrogenated lecithin, hydroxy lecithin, and the like.

Lecithin simply extracted and purified from a plant that contains unsaturated fatty acid groups can also be used. However, hydrogenated lecithin is preferable in terms of color tone and oxidative stability of the compound.

Ordinary lecithin extracted from plants contains phosphatidylcholine (PC), phosphatidylethanolamine (PE), phosphatidylinositol (PI), phosphatidylserine (PS), phosphatidic acid (PA), and the like in a certain ratio depending on the raw material. For example, soy lecithin is a mixture of 29% PC, 31% PE, 26% PI, 13% PA, and 1% PS. In order to modify the properties of the lecithin, fractionation of these components is commonly performed. In particular, a fractionation treatment is commonly performed to increase the content of phosphatidylcholine (PC), which can easily form a liposome structure. To reduce the size of the particles and stabilize the ceramide dispersion composition of the present invention, the content of phosphatidylcholine (PC) is preferably 50% or more, and particularly preferably 60% to 80%.

Examples of usable commercially available lecithin include SLP-White, SLP-White H, SLP-White Lyso H, SLP-PC35, SLP-PC70, SLP-PC70HS, SLP-PC92H, SLP-LPC70, and SLP-LPC70H, all of which are produced by Tsuji Oil Mills Co., Ltd.; Phospholipon 80H, Phospholipon 90G, and Phospholipon 90H, all of which are produced by Lipoid; Emulmetik-300, Emulmetik-320, Emulmetik-900, Emulmetik-930, and Emulmetik-950, all of which are produced by Lucas Meyer Cosmetics; Lecinol S-10, Lecinol S-10M, Lecinol S-10E, and Lecinol S-10EX, all of which are produced by Nikko Chemicals Co., Ltd.; Leciplart SOY75H and Leciplart SOY95H, both of which are produced by Technoble Co., Ltd.; and the like. Of these, SLP-PC70, SLP-PC70H, and SLP-LPC70H are particularly preferable.

The glycerophospholipids, including the compounds and lecithins described above, may be used singly or in a combination of two or more.

Sphingophospholipids, like all sphingolipids, have a ceramide (sphingosine in amide linkage with a fatty acid) structure and also have a phosphobase as a hydrophilic head. Those in which the base is choline are called "sphingomyelin." Those in which the base is ethanolamine are called "ceramide phosphoethanolamine."

Sphingomyelin and ceramide phosphoethanolamine, for example, can be preferably used in the ceramide dispersion composition of the present invention.

Examples of raw materials marketed as sphingomyelin or products containing sphingomyelin include high-purity sphingomyelin COATSOME NM series (produced by NOF corporation), Milk Ceramide MC-5 (produced by Megmilk Snow Brand Co., Ltd.), Ceramide CK (derived from chickens; produced by Genuine R&D Co., Ltd.), and Ceramide BT (derived from buttermilk; produced by Genuine R&D Co., Ltd.). These can also be used.

As the phospholipid used in the ceramide dispersion composition of the present invention, one or more glycerophospholipids alone or one or more sphingophospholipids alone may be used, or a combination of these may be used. A combination of these is preferable. When one or more glycerophospholipids and one or more sphingophospholipids are used in combination, the mass ratio of the glycerophospholipid(s) to the sphingophospholipid(s) is particularly preferably 9:1 to 5:5.

The phospholipid content in the ceramide dispersion composition of the present invention is preferably 0.01 to 10 parts by mass, per part by mass of the nonionic surfactant, in terms of reduction in the size of the dispersed particles and a skin care effect when the ceramide dispersion composition is used for cosmetics etc. The lower limit of this range may be 0.05, 0.1, 0.15, 0.2, 0.25, or 3 parts by mass. The upper limit of this range may be 9, 8, 7, 6, 5, 4.5, or 4 parts by mass. This range is more preferably 0.1 to 5 parts by mass, and even more preferably 0.2 to 2 parts by mass.

The ceramide dispersion composition of the present invention may further preferably comprise a sterol compound. Incorporating a sterol compound into the composition is preferable because it can further improve the skin care effect when the ceramide dispersion composition of the present invention is used as a cosmetic.

Examples of sterol compounds include cholesterol and phytosterol. Cholesterol is preferable.

Although there is no particular limitation, cholesterol obtained by purifying crude cholesterol obtained as the main component of lanolin by extraction is preferably used. Commercially available products may be used as cholesterol. Examples of the commercially available products include Cholesterol JSQI (produced by Nippon Fine Chemical Co., Ltd.), Cholesterol (produced by Croda Japan), Riken Cholesterol (produced by Riken Vitamin Co., Ltd.), and the like. In the ceramide dispersion composition of the present invention, the cholesterol content is preferably 0.1 to 3 parts by mass, more preferably 0.3 to 2 parts by mass, and even more preferably 0.5 to 1.5 parts by mass, per part by mass of the naturally occurring free ceramide.

The ceramide dispersion composition of the present invention is preferably in the form in which an oil phase is dispersed in an aqueous phase (e.g., an oil-in-water (O/W) emulsion). In this case, the naturally occurring free ceramide, the C₂₋₆ diol, and the nonionic surfactant are preferably present in the oil phase, and the phospholipid is preferably present in the aqueous phase. When the composition contains a sterol compound, the sterol compound is preferably present in the oil phase.

When the composition is in the form of an O/W emulsion, the average particle size of the oil droplets (i.e., dispersed particles) is preferably 100 nm or less. The average particle size of the dispersed particles is preferably 50 nm or less, more preferably 30 nm or less, and even more preferably 20 nm or less, in terms of stability during storage and a skin care effect. The lower limit of the average particle size of the dispersed particles is not particularly limited and may be, for example, 1 nm or more.

The average particle size of the dispersed particles in the present invention means the volume average particle size measured using the dynamic light scattering method.

Examples of commercially available measurement devices that can measure the average particle size by the dynamic light scattering method include a Nanotrac Wave particle size distribution analyzer (MicrotracBEL Corp.), Zetasizer Ultra (produced by Malvern Panalytical), a FPAR-1000 fiber-optics particle analyzer (produced by Otsuka Electronics Co., Ltd), and the like.

In the present specification, the volume average particle size of the dispersed particles can be measured using, for example, a dynamic-light-scattering Nanotrac Wave (produced by MicrotracBEL Corp.), and can be specifically measured as follows. A sample taken from the ceramide dispersion composition of the present invention is diluted with pure water so that the concentration of ceramide contained in the sample is 0.1 mass%, and measurement is performed using a quartz cell. The average particle size is determined as the volume average particle size (Mv) by setting the sample refractive index to 1.600 and the dispersion medium refractive index to 1.333 (pure water), and using the viscosity of pure water as the viscosity of the dispersion medium.

The average particle size of the dispersed particles can also be adjusted by suitably adjusting factors such as high-pressure emulsification and dispersion conditions (number of passes, pressure, and temperature), stirring conditions (shearing force and temperature), and the ratio of the oil phase to the aqueous phase in the production method, in addition to the components of the dispersion composition.

The ceramide dispersion composition of the present invention may comprise other components as long as the effects of the composition are not impaired.

The oil phase may contain other components to enhance the skin care feeling when the composition is used as a cosmetic. Examples of the components include oils and fats, higher fatty acids, higher alcohols, hydrophobic functional components, and the like.

Examples of oils and fats include hydrocarbon-based oils and fats, such as squalane, hydrogenated polyolefin, Vaseline, and liquid paraffin; vegetable oils and fats, such as jojoba oil, avocado oil, coconut oil, palm oil, olive oil, and macadamia nut oil; synthetic ester oils, such as ethylhexyl palmitate, octyldodecyl myristate, isopropyl myristate, and isononyl isononanoate; and the like.

Examples of higher fatty acids include saturated or unsaturated fatty acids having 10 to 24 (10, 11, 12, 13, 14, 15, 16, 17, 18, 19, 20, 21, 22, 23, or 24) carbon atoms. Specific examples include lauric acid, myristic acid, palmitic acid, stearic acid, behenic acid, isostearic acid, oleic acid, linoleic acid, and the like.

Examples of higher alcohols include cetyl alcohol, stearyl alcohol, cetostearyl alcohol, behenyl alcohol, and the like.

Various hydrophobic functional components can be used. Examples of components that have, for example, an antioxidant effect include tocopherol, tocotrienol, carotenoids, ascorbic acid derivatives, various polyphenols, and the like.

In addition to preservatives, fragrances, water-soluble pigments, pH adjusters such as acids and alkalis, salts having a pH-buffering effect, and the like, it is possible to add, for example, various thickening polymers to the aqueous phase as agents for improving the skin-care feeling. Examples of thickening polymers include natural polymers, such as sodium hyaluronate, xanthan gum, gum arabic, guar gum, ghatti gum, gellan gum, carrageenan, collagen, pectin, and Aphanothece sacrum polysaccharides; synthetic polymers typified by carbomer; and the like.

The ceramide dispersion composition of the present invention can be obtained, for example, by a production method comprising mixing a dispersed phase component (oil phase component) containing the naturally occurring free ceramide, and a continuous phase component (aqueous phase component).

The method of mixing the aqueous phase component and the dispersed phase component is not particularly limited. They may be mixed by using, for example, a known method, such as an ultrasonic dispersion method, a high-pressure emulsification method, or a jet injection method in which the dispersed phase component is directly injected into the continuous phase component.

A preferred example of the method for producing the ceramide dispersion composition of the present invention is a method comprising heating an oil phase component containing the naturally occurring free ceramide, the C₂₋₆ diol, and the nonionic surfactant in combination, mixing the oil phase component with an aqueous phase component containing the phospholipid, and subjecting the mixture to a dispersion treatment to obtain a coarse dispersion (also referred to below as "preliminary dispersion treatment step"); and subjecting the coarse dispersion to a dispersion treatment by using an ultrasonic dispersion method or a high-pressure emulsification method (also referred to below as "main dispersion treatment step"), wherein dispersed particles containing the naturally occurring free ceramide with an average particle size of 100 nm or less, which is a fine particle size, are formed.

The production method of this embodiment is described in more detail below.

In the preparation of the coarse dispersion in the preliminary dispersion treatment step, an oil phase component containing the naturally occurring free ceramide, the C₂₋₆ diol, the nonionic surfactant, and one or more other hydrophobic components (e.g., a sterol compound) is heated for dissolution and then mixed with an aqueous phase component containing the phospholipid, and the mixture is subjected to a dispersion treatment to obtain a coarse dispersion in which dispersed particles (oil phase) containing the naturally occurring free ceramide in a molten state are coarsely dispersed in the continuous phase (aqueous phase).

In the oil phase component, the naturally occurring free ceramide, the C₂₋₆ diol, the nonionic surfactant, and the other hydrophobic component(s) may simply be mixed. The mixing method is not particularly limited, and examples include a method of mixing by stirring.

Examples of the other hydrophobic components include components such as cholesterol, phytosterol, polyethylene glycol, and higher fatty acids.

The aqueous phase component contains the phospholipid (e.g., lecithin). The phospholipid is added to water with stirring and dispersed uniformly with stirring and heating. For stirring, for example, a magnetic stirrer, a household mixer, a paddle mixer, an impeller mixer, a homomixer, a disperser mixer, or an ultramixer may be used as a stirrer. To accelerate homogenization, a mixer capable of high-speed stirring is preferable.

Moreover, water-soluble components other than the phospholipid can be added to the aqueous phase component. Examples include polyhydric alcohols such as glycerol, salts, pH adjusters, water-soluble polymers, and the like.

The means for obtaining the coarse dispersion by mixing the oil phase component and the aqueous phase component and subjecting the mixture to a dispersion treatment is not particularly limited, and a common stirrer can be used. Examples of stirrers include magnetic stirrers, household mixers, paddle mixers, impeller mixers, homomixers, disperser mixers, ultramixers, and the like.

The time of the preliminary dispersion treatment is not particularly limited, and can be appropriately set according to, for example, the type of stirrer and the composition of the liquid before the dispersion treatment.

The main dispersion treatment step may be performed, for example, by subjecting the coarse dispersion obtained in the preliminary dispersion treatment (water or the like may be further mixed with the coarse dispersion, if necessary) to a dispersion treatment using an ultrasonic dispersion method (referred to below as "ultrasonic dispersion treatment") or a dispersion treatment using a high-pressure emulsification method (referred to below as "high-pressure emulsification treatment").

In the main dispersion treatment step, the coarse dispersion containing the naturally occurring free ceramide (preferably in a molten state) (water or the like may be further mixed with the coarse dispersion, if necessary) is subjected to a dispersion treatment to obtain a ceramide dispersion composition comprising dispersed particles containing the naturally occurring free ceramide.

The dispersion treatment in the main dispersion step is preferably a high-pressure emulsification treatment, in terms of reducing the size of the dispersed particles.

The high-pressure emulsification treatment refers to a dispersion treatment in which a shearing force of 10 MPa or more is applied to a material to be dispersed. To reduce the size of the dispersed particles, the shearing force applied to the material to be dispersed is preferably 100 MPa or more, and more preferably 150 MPa or more. The upper limit is preferably, for example, 300 MPa or less in terms of temperature increase and pressure resistance.

The means for the high-pressure emulsification treatment is not particularly limited, and a general high-pressure emulsification device can be used. Examples of high-pressure emulsification devices include high-pressure homogenizers, such as Star Burst HJP-25005 (produced by Sugino Machine Limited), Microfluidizer (produced by Microfluidics), Nano-Mizer (produced by Yoshida Kikai Co., Ltd.), a Gaulin-type homogenizer (produced by APV), a Rannie-type homogenizer (produced by Rannie), a high-pressure homogenizer (produced by Niro Soavi), a homogenizer (produced by Sanwa Machinery Trading Co., Ltd.), a high-pressure homogenizer (produced by Izumi Food Machinery), and an ultra-high-pressure homogenizer (produced by IKA) .

The temperature during the high-pressure emulsification treatment is preferably set to 30 to 80°C, and more preferably 40 to 70°C.

Although the high-pressure emulsification treatment may be performed once, the high-pressure emulsification treatment is preferably performed two or more times, and more preferably two to five times, in order to increase the homogeneity of the entire liquid. To maintain the particle size of the dispersed particles, it is preferable that the emulsified liquid, which is a composition that has undergone emulsification and dispersion, is cooled through some sort of cooler within 30 seconds, and preferably within 3 seconds, immediately after passing through the chamber.

The dispersion treatment in the main dispersion step may be an ultrasonic dispersion treatment. To further increase the dispersion effect, it is also preferable to perform an ultrasonic dispersion treatment before subjecting the coarse dispersion to the high-pressure emulsification treatment. For the dispersion treatment by ultrasonic application, a general ultrasonic dispersion device may be used.

Examples of ultrasonic dispersion devices include ultrasonic homogenizers US-150T, US-600T, US-1200T, RUS-1200T, and MUS-1200T (produced by Nippon Seiki Co., Ltd.), ultrasonic processors UIP2000, UIP-4000, UIP-8000, and UIP-16000 (produced by Hielscher), and the like. These ultrasonic dispersion devices may be used at a frequency of 25 kHz or less, and preferably 15 to 20 kHz.

The production method may comprise other steps as necessary, in addition to the preliminary dispersion treatment step and the main dispersion treatment step. Examples of the other steps include a heat sterilization step and the like.

In the present specification, the term "comprising" includes "consisting essentially of" and "consisting of." The present invention covers all combinations of the elements described in the present specification.

In addition, the various characteristics (properties, structures, functions, etc.) described in each embodiment of the present invention described above may be combined in any way in specifying the subject matter included in the present invention. In other words, the present invention includes all the subject matter comprising all combinations of the combinable characteristics described in the present specification.

### Examples

### Preparation of Ceramide Dispersion Compositions

Genuine Ceramide WSS (produced by Genuine R&D Co., Ltd.; free ceramide extracted from soy sauce lees produced in Fukuoka Prefecture) was used as a ceramide composition for preparing a ceramide dispersion composition. This composition is a ceramide composition obtained by subjecting soy sauce lees to ethanol extraction, and purifying the high-purity free ceramides by solvent fractionation, with reference to the method described in JP2012-126910A. More specifically, the ceramide composition was prepared in the following manner. A dried product of pressed lees, which is a by-product of soy sauce, was subjected to ethanol extraction, and the obtained extract was subjected to solid-liquid separation using ethanol and water to obtain solids. Further, the solids were washed with acetone, ethanol, and water; dried; and pulverized, followed by washing with water, acetone, and hexane. Ethanol extraction was then performed again to obtain solids as a ceramide composition. The ceramide content of the lot used was 98.4%.

The molecular species composition of Genuine Ceramide WSS was determined by a LCMS-IT-TOF method. Specifically, each molecule was separated using a TSKgelODS-100Z (Nacalai Tesque, Inc.) as an LC column, and the molecular species were determined using an LCMS-IT-TOF apparatus (produced by Shimadzu Corporation) as a detector and an APCI as an ionization probe. MS-MS analysis identified each of the sphingoid bases and fatty acids.

The classification of free ceramide species based on the composition of the sphingoid base skeleton was as follows: ceramide AP = 92.7%, ceramide NP = 1.3%, and another ceramide (dihydroxylignoceroyl phytosphingosine) = 6.0%. The percentage of phytoceramides in the total ceramides was almost 100%.

The sum of carbon atoms of the sphingoid base and the fatty acid was as follows: C38 = 0.4%, C39 = 0.4%, C40 = 8.2%, C41 = 3.2%, C42 = 67.2%, C43 = 7.8%, C44 = 12.3%, and C45 = 0.5%. The percentage of free ceramides having 40 or more carbon atoms was almost 99% of the total free ceramides, and the percentage of free ceramides having 42 or more carbon atoms was about 88% of the total free ceramides.

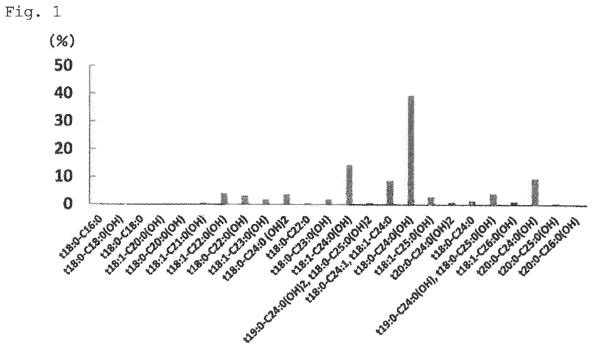

Fig. 1 shows the percentages (mass%) of various free ceramide species in the ceramide composition found from the analysis. Three free ceramide species, i.e., t18:0-24:0h, t18:1-24:0h, and t20:0-24:0h, made up not less than 50 mass% of the ceramide composition. It was also found that the ceramide composition contains other free ceramides.

### Example 1

Liquid A and liquid B having the following compositions were prepared and used for preparing ceramide dispersion compositions. In this example, "parts" denotes parts by mass.

**Liquid A**

| | |
|---|---|
| Genuine Ceramide WSS | 1.0 part |
| 1,3-Butylene glycol | 10.0 parts |
| Decaglycerol monoisostearate | 3.0 parts |
| (Decaglyn 1-ISV) | |

**Liquid B**

| | |
|---|---|
| Lecithin (SLP-PC70) | 1.0 part |
| Phenoxyethanol | 0.2 parts |
| Glycerol | 26.9 parts |
| Water | 57.9 parts |

Liquid A was prepared by dissolving 15 g of the raw materials in the proportions described above by stirring while warming at 130°C or less. Liquid B was prepared by mixing 85 g of the raw materials in the proportions described above and stirring the mixture with a homomixer (produced by AS ONE Corporation) at 70°C for 30 minutes to homogenize it. Liquid A was cooled to 100°C, and homogenized liquid B was added to liquid A, followed by dispersing at 4000 rpm for 15 minutes with Polytron (produced by Polytron), thereby obtaining a pre-dispersion. Subsequently, the obtained pre-dispersion was cooled to 60°C and then subjected to a high-pressure emulsification (dispersion) treatment at a pressure of 150 MPa with Star Burst HJP-25005 (produced by Sugino Machine Limited), thereby obtaining a ceramide dispersion composition (Example 1).

### Examples 2 to 6, 10, 11, 13, 14, 15 to 17, and 21 to 24, and Comparative Examples 7 to 9, 12, 18 to 20, 29, and 30

The ceramide dispersion compositions of these Examples and Comparative Examples were obtained in the same manner as in Example 1, except that liquids A and B having the compositions shown in Tables 1 and 2 were used. In Tables 1 and 2, "parts," which is the unit of the amount of each component, denotes parts by mass.

### Example 25

The ceramide dispersion composition of Example 25 was obtained in the same manner as in Example 1, except that the high-pressure emulsification treatment was performed at a pressure of 100 MPa.

### Example 26

The ceramide dispersion composition of Example 26 was obtained in the same manner as in Example 1, except that the high-pressure emulsification treatment was performed at a pressure of 50 MPa.

### Example 27

The ceramide dispersion composition of Example 27 was obtained in the same manner as in Example 1, except that the time of dispersion using Polytron was 10 minutes, and that the high-pressure emulsification treatment was not performed.

### Example 28

The ceramide dispersion composition of Example 28 was obtained in the same manner as in Example 1, except that an ultrasonic emulsification treatment was performed instead of the high-pressure emulsification treatment. The ultrasonic emulsification treatment was performed by ultrasonic irradiation for 4 minutes with stirring using an US-150T ultrasonic homogenizer (produced by Nippon Seiki Co., Ltd.).

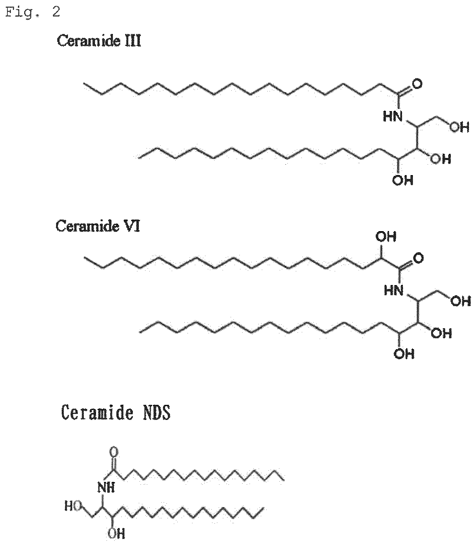

The details of the materials used in the Examples and Comparative Examples are as follows. Fig. 2 shows the structural formulas of Ceramide-III, Ceramide-VI, and Ceramide NDS. As can be seen from Fig. 2, the number of carbon atoms in the ceramide skeleton of each of these three free ceramides is 36; i.e., the fatty acid has 18 carbon atoms, and the sphingoid base has 18 carbon atoms.

Ceramide-III: Ceramide III (produced by Evonik Industries AG)
Ceramide-VI: Ceramide VI (produced by Evonik Industries AG)
Ceramide NDS: TIC-001 (produced by Takasago International Corporation)
Cholesterol: Riken Cholesterol (produced by Riken Vitamin Co., Ltd.)
1,3-Butanediol: Haisugarcane BG (produced by Kokyu Alcohol Kogyo Co., Ltd.)
Ethylene glycol: Guaranteed reagent of Wako Pure Chemical Industries, Ltd.
Propylene glycol: Guaranteed reagent of Wako Pure Chemical Industries, Ltd.
Pentylene glycol: Green Pentanediol (GSI Creos Corporation)
Hexylene glycol: Guaranteed reagent of Wako Pure Chemical Industries, Ltd.
Diethylene glycol: Guaranteed reagent of Wako Pure Chemical Industries, Ltd.
Ethanol: Fermented alcohol (produced by Japan Alcohol Corporation)
n-Butanol: Guaranteed reagent of Wako Pure Chemical Industries, Ltd.
Decaglycerol monoisostearate:
   NIKKOL Decaglynl-ISV (produced by Nikko Chemicals Co., Ltd.)
   NIKKOL Decaglyn1-ISVEX (produced by Nikko Chemicals Co., Ltd.) Polysorbate 60: NIKKOL TS-10V (produced by Nikko Chemicals Co., Ltd.)
   HCO-60 (PEG60 hydrogenated castor oil): NIKKOL HCO60 (produced by Nikko Chemicals Co., Ltd.)
Lecithin:
   SLP-PC70 (produced by Tsuji Oil Mills Co., Ltd.)
   SLP-PC70H (produced by Tsuji Oil Mills Co., Ltd.)
   SLP-LPC70H (produced by Tsuji Oil Mills Co., Ltd.)
   SLP-White (produced by Tsuji Oil Mills Co., Ltd.)
Sphingomyelin: Ceramide BT (produced by Genuine R&D Co., Ltd.)
Glycerol: Triol VE (produced by Kokyu Alcohol Kogyo Co., Ltd.)
Phenoxyethanol: Phenoxyethanol SP (produced by Yokkaichi Chemical Co., Ltd.)

### Evaluation of Dispersion Compositions

### (1) Evaluation of Stability during Storage

The particle size of the dispersed particles in the ceramide dispersion composition obtained in each of the Examples and Comparative Examples immediately after preparation and after elapse of a time was measured, and the stability over time was evaluated as follows. Tables 1 and 2 show the results.

### (1-1) Particle Size of Dispersed Particles in Ceramide Dispersion Compositions Immediately after Preparation

The particle size (volume average particle size) of the dispersed particles in each ceramide dispersion composition immediately after preparation was measured using a dynamic-light-scattering Nanotrac Wave (produced by MicrotracBEL Corp.). The measurement of the volume average particle size was performed by diluting a sample taken from the ceramide dispersion composition with pure water so that the concentration of ceramide contained in the sample was 0.1 mass%. The volume average particle size (Mv) was determined by setting the sample refractive index to 1.600 and the dispersion medium refractive index to 1.333 (pure water), and using the viscosity of pure water as the viscosity of the dispersion medium.

### (1-2) Particle Size of Dispersed Particles in Ceramide Dispersion Compositions after Storage

After each ceramide dispersion composition was stored in a thermostatic chamber at 40°C for 60 days, the composition was cooled back to about 25°C, and the particle size of the dispersed particles was measured in the same manner as for the ceramide dispersion compositions immediately after preparation.

The results in Tables 1 and 2 show that the ceramide dispersion composition in each of the Examples had a small particle size immediately after preparation and high stability with a small change in the particle size during storage at 40°C.

Although even the ceramide dispersion compositions prepared using non-natural human-type free ceramides (Comparative Examples 18 to 20) had a small particle size immediately after preparation and relatively good storage stability as in the ceramide dispersion compositions of the Examples, there was a clear difference in the evaluation as a cosmetic.

**Table 1**

| Ceramide dispersion composition | | | 1 | 2 | 3 | 4 | 5 | 6 | 7 | 8 | 9 | 10 | 11 | 12 | 13 | 14 |
|---|---|---|---|---|---|---|---|---|---|---|---|---|---|---|---|---|
| | | | Ex | Ex. | Ex. | Ex. | Ex. | Ex. | Comp. Ex. | Comp. Ex. | Comp. Ex. | Ex | Ex. | Comp. Ex. | Ex. | Ex. |
| Proportion (parts) | Liquid A | Genuine Ceramide WSS | 1.0 | 1.0 | 1.0 | 1.0 | 1.0 | 1.0 | 1.0 | 1.0 | 1.0 | 1.0 | 1.0 | 1.0 | 1.0 | 1.0 |
| | | Cholesterol | - | - | - | - | - | - | - | - | - | - | - | - | - | 1.0 |
| | | Butylene glycol (1,3-butanediol) | 10.0 | - | - | - | - | - | - | - | 2.0 | 3.0 | 20.0 | 30.0 | 10.0 | 10.0 |
| | | Ethylene glycol | - | 10.0 | - | - | - | - | - | - | - | - | - | - | - | - |
| | | Propylene glycol | - | | 10.0 | - | - | - | - | - | - | - | - | - | - | - |
| | | Pentylene glycol (1,2-pentanediol) | - | - | - | 10.0 | - | - | - | - | - | - | - | - | - | - |
| | | Hexylene glycol (1,2-hexanediol) | - | - | - | - | 10.0 | - | - | - | - | - | - | - | - | - |
| | | Diethylene glycol | - - | - - | - - | - - | - - | 10.0 | - | - | - | - | - | - | - | - |
| | | Ethanol glycol | - | - | - | - | - | - | 10.0 | - | - | - | - | - | - | - |
| | | n-Butanol | - | - | - | - | - | - | - | 10.0 | - | - | - | - | - | - |
| | | Decaglycerol monoisostearate (Decaglyn 1-ISV) | 3.0 | 3.0 | 3.0 | 3.0 | 3.0 | 3.0 | 3.0 | 3.0 | 3.0 | 3.0 | 3.0 | 3.0 | 3.0 | 3.0 |
| | Liquid B | Lecithin (SLP-PC70) | 1.0 | 1.0 | 1.0 | 1.0 | 1.0 | 1.0 | 1.0 | 1.0 | 1.0 | 1.0 | 1.0 | 1.0 | 1.0 | 1.0 |
| | | Glycerol | 26.9 | 26.9 | 26.9 | 26.9 | 26.9 | 26.9 | 26.9 | 26.9 | 34.9 | 33.9 | 16.9 | 6.9 | | 25.9 |
| | | Phenoxyethanol | 0.2 | 0.2 | 0.2 | 0.2 | 0.2 | 0.2 | 0.2 | 0.2 | 0.2 | 0.2 | 0.2 | 0.2 | 0.5 | 0.2 |
| | | Water | 57.9 | 57.9 | 57.9 | 57.9 | 57.9 | 57.9 | 57.9 | 57.9 | 57.9 | 57.9 | 57.9 | 57.9 | 84.5 | 57.9 |
| Dispersion conditions | Dispersion method | | High pressure | High pressure | High pressure | High pressure | **High** pressure | High pressure | High pressure | High pressure | High pressure | High pressure | High pressure | High pressure | High pressure | High pressure |
| | High-pressure emulsification dispersion pressure (MPa) | | 150 | 150 | 150 | 150 | 150 | 150 | 150 | 150 | 150 | 150 | 150 | 150 | 150 | 150 |
| Characteristic value | Diol/WSS ratio | | 10 | 10 | 10 | 10 | 10 | 10 | 10 | 10 | 2 | 3 | 20 | 30 | 10 | 10 |
| | Percentage (%) of those in which the number of carbon atoms of ceramide molecule is 40 or more | | 99 | 99 | 99 | 99 | 99 | 99 | 99 | 99 | 99 | 99 | 99 | 99 | 99 | 99 |
| | Percentage (%) of those in which the number of carbon atoms of ceramide molecule is 42 or more | | 88 | 88 | 88 | 88 | 88 | 88 | 88 | 88 | 88 | 88 | 88 | 88 | 88 | 88 |
| Dispersion evaluation | Particle size immediately after preparation Mv (nm) | | 7 | 35 | 10 | 15 | 44 | 13 | × | × | × | 56 | 8 | 89 | 8 | 9 |
| | Particle size after 60 days at 40°C Mv(nm) | | 11 | 72 | 15 | 20 | 87 | 21 | × | × | × | 89 | 25 | 212 | 16 | 10 |

| | | | | | | | | | | | | | | | | |
|---|---|---|---|---|---|---|---|---|---|---|---|---|---|---|---|---|
| * The symbol "×" indicates that, due to the presence of insoluble matter (aggregated floating matter), the particle size could not be measured. | | | | | | | | | | | | | | | | |

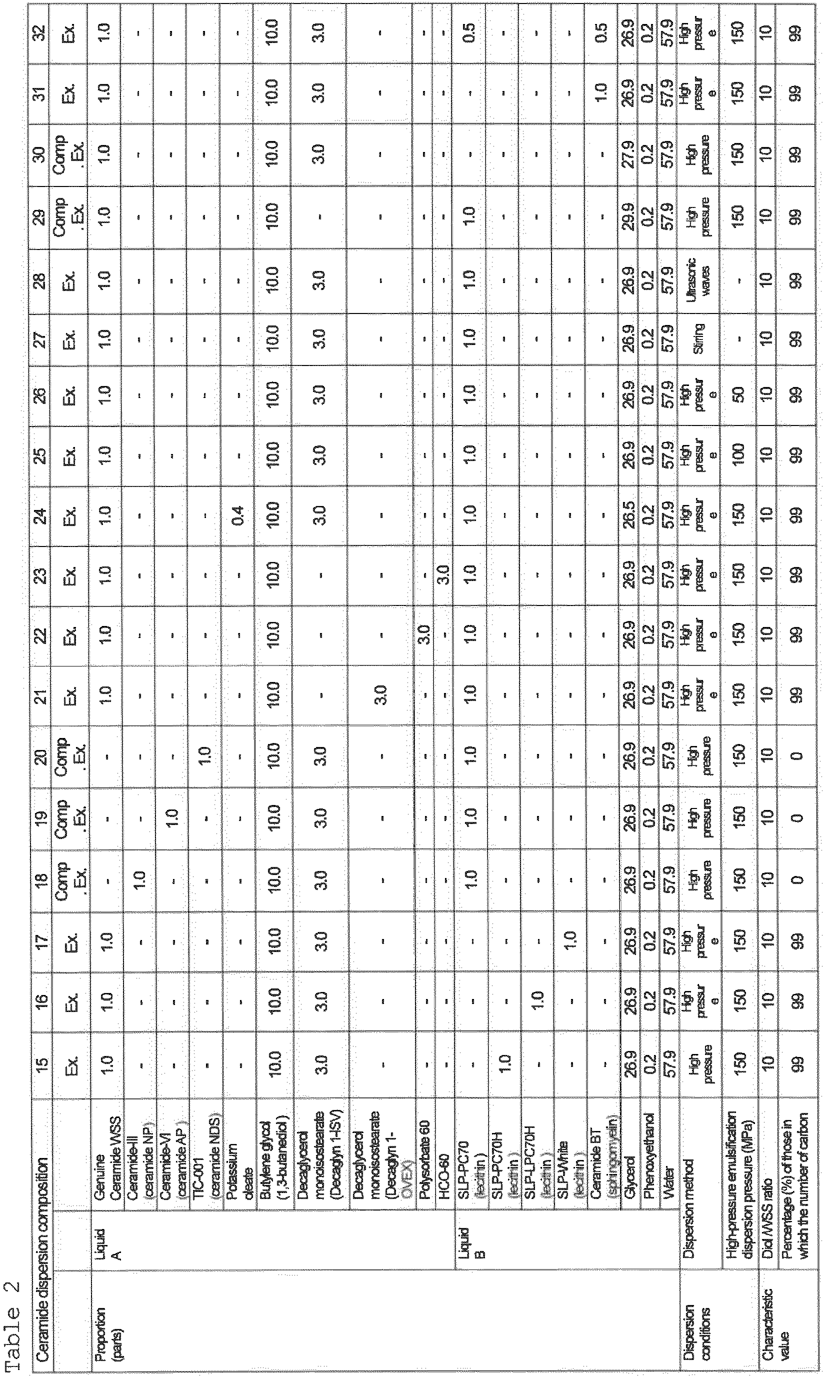

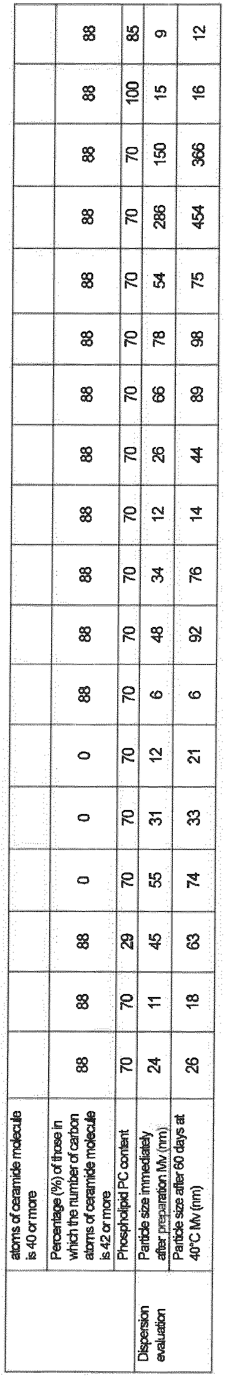

### (2) Skin Care Effect

Among the ceramide dispersion compositions shown in Tables 1 and 2, the compositions of Examples 1, 14, and 24 and Comparative Examples 18 to 20 were evaluated for their skin care effect when used as a cosmetic, as described below. Table 3 shows the results. As a ceramide blank, a composition prepared by the same process as in Example 1 except that only the ceramide composition (Genuine Ceramide WSS) was removed (Comparative Example 31) was also evaluated.

Each ceramide dispersion composition was individually applied to the back of the left hand of each of five subjects with significantly rough dry skin on the hands by spreading it over the entire surface of the back of the left hand twice a day for two weeks. Compared with the back of the right hand, to which the composition was not applied, "no change" was given one point, and "the state in which skin roughness was no longer observed" was given five points. The degree of improvement after two weeks was evaluated on a five-point scale, and the average score of the five subjects was calculated.

**Table 3**

| Ceramide dispersion composition | 1 | 14 | 24 | 18 | 19 | 20 | 31 |
|---|---|---|---|---|---|---|---|
| | Ex. | Ex. | Ex. | Comp. Ex. | Comp. Ex. | Comp. Ex. | Comp. Ex. |
| Ceramide | WSS | WSS | WSS | Cerlll | CerVI | CerNDS | - |
| Cholesterol | - | Contained | - | - | - | - | - |
| Fatty acid | - | - | Contained | - | - | - | - |
| Skin care effect | 4.2 | 4.6 | 4.4 | 2.6 | 2.4 | 2.4 | 1.4 |

### Results of Skin Care Evaluation

As shown in Table 3, the dispersion compositions of Comparative Examples 18 to 20, which use the non-natural human-type free ceramides, had a skin care effect compared with the composition prepared without using a ceramide; however, the skin care effect of the dispersion compositions of Comparative Examples 18 to 20 was greatly inferior to that of the dispersion compositions prepared using the naturally occurring human-type free ceramide.

## Claims

1. A ceramide dispersion composition comprising a naturally occurring free ceramide, a nonionic surfactant, a phospholipid, and a C₂₋₆ diol,
the mass ratio of the naturally occurring free ceramide to the diol being 1:3 to 25,
the naturally occurring free ceramide comprising a free ceramide that contains a fatty acid having 20 or more carbon atoms in the ceramide skeleton thereof.

2. The ceramide dispersion composition according to claim 1, wherein 90 mass% or more of the naturally occurring free ceramide is a free ceramide in which the number of carbon atoms constituting a molecule is 40 or more, and 60 mass% or more of the naturally occurring free ceramide is a free ceramide in which the number of carbon atoms constituting a molecule is 42 or more.

3. The ceramide dispersion composition according to claim 1 or 2, wherein 60 mass% or more of the naturally occurring free ceramide is ceramide AP and ceramide NP.

4. The ceramide dispersion composition according to any one of claims 1 to 3, wherein 80 mass% or more of the naturally occurring free ceramide is ceramide AP.

5. The ceramide dispersion composition according to any one of claims 1 to 4, wherein the naturally occurring free ceramide is at least one free ceramide selected from the group consisting of
free ceramides with a ceramide skeleton that is a combination of a sphingoid base containing 3 hydroxyl groups, 18 carbon atoms, and 0 or 1 carbon-carbon double bond; and a fatty acid containing 22 to 26 carbon atoms, 0 carbon-carbon double bonds, and 0, 1, or 2 hydroxyl groups, and
free ceramides with a ceramide skeleton that is a combination of a sphingoid base containing 3 hydroxyl groups, 20 carbon atoms, and no carbon-carbon double bonds; and a fatty acid containing 24 or 25 carbon atoms, 0 carbon-carbon double bonds, and 0, 1, or 2 hydroxyl groups.

6. The ceramide dispersion composition according to any one of claims 1 to 5, wherein the C₂₋₆ diol is at least one diol selected from the group consisting of propylene glycol, butylene glycol, diethylene glycol, and pentylene glycol.

7. The ceramide dispersion composition according to any one of claims 1 to 6, wherein the phospholipid is at least one phospholipid selected from the group consisting of glycerophospholipids and sphingophospholipids.

8. The ceramide dispersion composition according to any one of claims 1 to 7, wherein 50 mass% or more of the phospholipid is phosphatidylcholine.

9. The ceramide dispersion composition according to any one of claims 1 to 8, wherein the nonionic surfactant is a polyglycerol mono-fatty acid ester.

10. The ceramide dispersion composition according to any one of claims 1 to 9, further comprising a sterol compound.

11. The ceramide dispersion composition according to any one of claims 1 to 10, further comprising a C₁₀₋₂₄ saturated or unsaturated fatty acid.

12. The ceramide dispersion composition according to any one of claims 1 to 11, wherein particles containing a naturally occurring human-type free ceramide and dispersed in the composition have an average particle size of 100 nm or less.

13. The ceramide dispersion composition according to any one of claims 1 to 12, which is a cosmetic composition or a food composition.
